(19) Europäisches Patentamt / European Patent Office / Office européen des brevets

(11) **EP 3 919 031 B1**

(12) **EUROPEAN PATENT SPECIFICATION**

(45) Date of publication and mention
of the grant of the patent:
**04.03.2026 Bulletin 2026/10**

(21) Application number: **20749082.2**

(22) Date of filing: **28.01.2020**

(51) International Patent Classification (IPC):
*A61F 13/0246* (2024.01)    *A61L 15/42* (2006.01)
*A61L 15/58* (2006.01)    *A61L 24/00* (2006.01)
*A61L 24/06* (2006.01)    *C08F 220/18* (2006.01)
*C08K 5/103* (2006.01)    *C09J 133/06* (2006.01)
*C09J 7/38* (2018.01)    *C09J 7/26* (2018.01)

(52) Cooperative Patent Classification (CPC):
(C-Sets available)
**A61F 13/0253; A61L 15/425; A61L 15/58;
A61L 24/0036; A61L 24/06; C08F 220/1809;
C09J 7/26; C09J 7/385; C09J 133/06;** C08K 5/103;
C09J 2301/122; C09J 2301/312; C09J 2301/408;
C09J 2400/243; C09J 2433/00      (Cont.)

(86) International application number:
**PCT/JP2020/002881**

(87) International publication number:
**WO 2020/158698 (06.08.2020 Gazette 2020/32)**

(54) **ADHESIVE SKIN PATCH MATERIAL**

HAUTPFLASTERMATERIAL

MATÉRIAU DE TIMBRE DERMIQUE ADHÉSIF

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **28.01.2019 JP 2019012196**

(43) Date of publication of application:
**08.12.2021 Bulletin 2021/49**

(73) Proprietor: **NITTO DENKO CORPORATION
Ibaraki-shi
Osaka 567-8680 (JP)**

(72) Inventors:
• **YOSHIDA, Noboru**
  **Ibaraki-shi, Osaka 567-8680 (JP)**
• **KODAMA, Kiyoaki**
  **Ibaraki-shi, Osaka 567-8680 (JP)**
• **ABE, Eriko**
  **Ibaraki-shi, Osaka 567-8680 (JP)**
• **ARIMA, Ryuhei**
  **Ibaraki-shi, Osaka 567-8680 (JP)**

(74) Representative: **Grünecker Patent- und
Rechtsanwälte
PartG mbB
Leopoldstraße 4
80802 München (DE)**

(56) References cited:
EP-A1- 0 676 183      EP-A1- 2 799 472
EP-A1- 3 498 245      WO-A1-2018/030447
JP-A- H0 838 544      JP-U- H 045 807

(52) Cooperative Patent Classification (CPC): (Cont.)

C-Sets
**A61L 15/58, C08L 33/08;
A61L 24/06, C08L 33/08;
C08F 220/1809, C08F 220/06, C08F 220/281;
C09J 133/06, C08K 5/103**

# EP 3 919 031 B1

**Description**

Technical Field

The present invention relates to an adhesive skin patch.

Background Art

A device (wearable device) that is mounted on a body of a user is conventionally known. The wearable device is versatile, and one example of the use is measurement of information such as blood glucose level, heart rhythm, myoelectricity, body temperature and the like of a user.

Furthermore, there are various manners to mount a wearable device. For example, a device mounting by sticking to a body of a user is known. Such a device is excellent in that due to high adhesiveness to a body of a user, accuracy of information is high, and mounting is simple. For example, Patent Document 1 discloses a device for cardiac monitoring that is used by sticking to a skin.

Tape-like adhesive skin patch having adhesiveness is sometimes used to stick such a device to a skin. Examples of adhesive compositions, patches and bandages are disclosed in Patent Documents 2 to 5

Related Art

Patent Documents

Patent Document 1: JP-T-2015-530225 (the term "JP-T" as used herein means a published Japanese translation of a PCT patent application)
Patent Document 2: EP 0 676 183 A1
Patent Document 3: EP 3 498 245 A1
Patent Document 4: EP 2 799 472 A1
Patent Document 5: JP H04 5807 U

Summary of Invention

Problems to Be Solved by the Invention

Skin irritation sometimes occurs when an adhesive skin patch is being stuck to a skin. For example, an itch occurs on the skin depending on components of an adhesive, and inflammation sometimes occurs. Furthermore, when a wearable device harder than an adhesive skin patch has been mounted on a skin, the wearable device is pressed into the skin and thus impression or pain sometimes occurs on the skin.

In addition, since a skin flexibly stretches, a wearable device and an adhesive skin patch cannot follow expansion and contraction of the skin, and as a result, those may peel from the skin or discomfort (skin tightness) and pain may be felt.

The wearable device may be required to be mounted on the skin over a long period of time depending on its uses. Therefore, an adhesive skin patch that has less skin irritation, is difficult to peel from a skin and suppresses discomfort during sticking to the skin has been desired.

The present invention has been made in view of the above and has an object to provide an adhesive skin patch that has less skin irritation, is difficult to peel from a skin and suppresses discomfort during sticking to a skin.

Means for Solving the Problems

The adhesive skin patch of the present invention that solves the above problems is an adhesive skin patch including a substrate and an adhesive layer, wherein the substrate is formed of a foam sheet having breaking elongation of 100% or more, breaking strength of 10 N/20 mm or less, a density in the range of from 0.15 to 0.4 g/cm$^3$ and a thickness of 0.300 mm or more, the adhesive layer is formed on one side of the substrate, and the adhesive layer contains an acrylic copolymer and a liquid or pasty component at room temperature.

In one aspect of the adhesive skin patch of the present invention, another adhesive layer may be further provided on a surface on a side opposite the surface of the substrate having the adhesive layer.

In one aspect of the adhesive skin patch of the present invention, the liquid or pasty component at room temperature may contain a carboxylic acid ester.

In one aspect of the adhesive skin patch of the present invention, the carboxylic acid ester may contain a glycerin ester of a saturated fatty acid.

Effects of the Invention

According to the present invention, an adhesive skin patch that has less skin irritation, is difficult to peel from a skin and suppresses discomfort during sticking to a skin is provided.

Brief Description of Drawings

[FIG. 1]

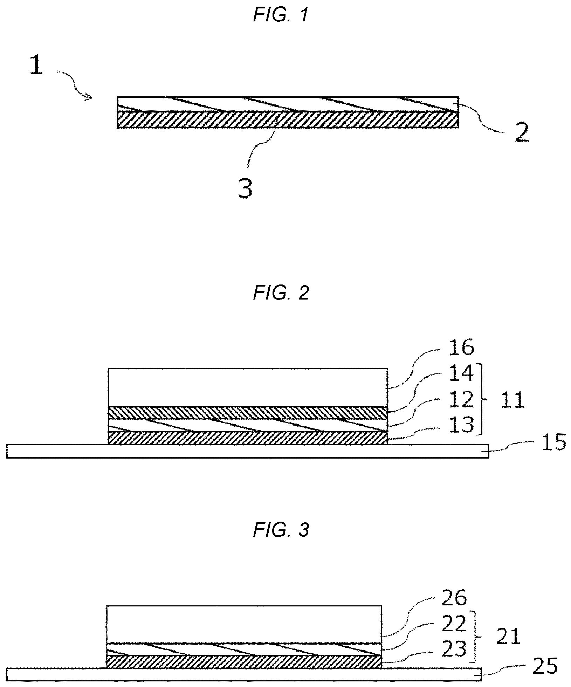

FIG. 1 is a schematic side view of an adhesive skin patch.
[FIG. 2]
FIG. 2 is a schematic view showing the state that a wearable device has been stuck to a skin using an adhesive skin patch in which both sides are an adhesive surface.
[FIG. 3]
FIG. 3 is a schematic view showing the state that a wearable device has been stuck to a skin using an adhesive skin patch in which only one side is an adhesive surface.

Embodiments for Carrying Out the Invention

The embodiments of the present invention have been described in detail blow. However, the present invention is not construed as being limited to the embodiments described below.

[Adhesive skin patch]

Schematic side view of the adhesive skin patch according to one aspect of the present embodiment is shown in FIG. 1. An adhesive skin patch 1 of the present embodiment has a substrate (support) 2 and an adhesive layer 3.
In the present embodiment, the substrate 2 is formed of a foam sheet having breaking elongation of 100% or more, breaking strength of 20 N/20 mm or less, a density in the range of from 0.15 to 0.4 g/cm$^3$ and a thickness of 0.300 mm or more. Therefore, the adhesive skin patch 1 of the present embodiment has high stretchability and stress relaxation (cushioning properties). For this reason, the adhesive skin patch 1 of the present embodiment can stretch following expansion and contraction of a skin, and peeling, discomfort (skin tightness) and skin irritation are difficult to occur.
Additionally, although the details are described hereinafter, due to high cushioning properties of the adhesive skin patch 1 of the present embodiment, a wearable device can be mounted on a skin in the state of less skin irritation.
In the present embodiment, the adhesive layer 3 is formed on one side of the substrate 2, and contains an acrylic copolymer and a liquid or pasty component at room temperature. Although the details are described hereinafter, the adhesive skin patch 3 has less skin irritation. In the following description, a surface of the substrate 2 on the side having the adhesive layer 3 provided thereon is sometimes described as a "first surface" and a surface on the side opposite the first surface is sometimes described as a "second surface".
In the adhesive skin patch of the present embodiment, only one side may be an adhesive surface and both surfaces may be an adhesive surface. In other words, the adhesive skin patch of the present embodiment may be provided with only an adhesive layer (hereinafter referred to as a "first adhesive layer") containing the above-described predetermined components, formed on the first surface of the substrate as an adhesive layer, and may be further provided with another adhesive layer (hereinafter referred to as a "second adhesive layer") formed on the second surface. The second adhesive layer may contain or may not contain the acrylic copolymer and the liquid or pasty component at room temperature.
As one aspect of the present embodiment, a schematic view showing the state that a wearable device 16 has been mounted on a skin 15 using an adhesive skin patch 11 provided with, as adhesive layers, a first adhesive layer 13 formed on a first surface of a substrate 12 and a second adhesive layer 14 formed on a second surface of the substrate, that is, the adhesive skin patch 11, both sides of which being an adhesive surface, is shown in FIG. 2. When the wearable device 16 is mounted on the skin using the adhesive skin patch 11, both sides of which being an adhesive surface, the first adhesive layer 13 containing the acrylic copolymer and the liquid or pasty component at room temperature is stuck to the skin 15 and the second adhesive layer 14 is stuck to the wearable device 16.
Thus, when the adhesive skin patch 11 of the present embodiment, both sides of which being an adhesive surface, has been used, the wearable device 16 can be mounted on the skin 15 with particularly difficult peeling and suppression of discomfort and skin irritation as described hereinafter, and this is preferred.

A wearable device is generally hard and cannot be stretched following expansion and contraction of a skin. As a result, peeling, skin tightness and pain due to this are difficult to be suppressed. On the other hand, when the wearable device 16 has been mounted on the skin using the adhesive skin patch 11 of the present embodiment as shown in FIG. 2, the substrate 12 having excellent cushioning properties is present between the skin 15 and the wearable device 16. As a result, stress due to the difference in stretchability between the skin 15 and the wearable device 16 can be relaxed, and peeling, skin tightness and pain due to the difference in stretchability can be suppressed.

When the wearable device is mounted on the skin using the conventional adhesive skin patch so as to cover the wearable device with the conventional adhesive skin patch, the wearable device is directly brought into contact with the skin so as to be pushed to the skin. As a result, impression and skin irritation such as pain occur on the skin. On the other hand, when the wearable device 16 has been mounted on the skin using the adhesive skin patch 11 of the present embodiment as shown in FIG. 2, the wearable device 16 is brought into contact with the skin 15 through the substrate 12 having excellent cushioning properties, and is not pushed on the skin 15. As a result, skin irritation is difficult to occur.

When the wearable device 26 is mounted on the skin using an adhesive skin patch 21, only one side of which being an adhesive surface, that is, the adhesive skin patch 21 that is not provided with the second adhesive layer, it is preferred that a first adhesive layer 23 is stuck to a skin 25 and the wearable device 26 is stuck to a second surface of a substrate 22 using an adhesive or a double-sided tape (not shown), as shown in FIG. 3. By mounting the wearable device on the skin like this, the same effect when the adhesive skin patch, both sides of which being an adhesive surface, is used can be exhibited.

Preferred aspect of the mounting method of the wearable device using the adhesive skin patch of the present embodiment is described above, but uses of the adhesive skin patch of the present embodiment are not limited to this. For example, the wearable device may be mounted on the skin so as to cover the wearable device with the adhesive skin patch of the present embodiment. Alternatively, the adhesive skin patch of the present embodiment can be used to protect a wound, like an adhesive plaster, or can be used to fix a bandage or gauze to the affected area, like a surgical tape.

To improve cushioning properties of the substrate, the thickness of the substrate is preferably large. Therefore, the thickness of the substrate (foam sheet) is 0.300 mm or more, preferably 0.600 mm or more and more preferably 0.900 mm or more.

On the other hand, in case where the thickness of the substrate is too large, there is a possibility that the substrate is easy to break or is easy to shift. Therefore, the thickness of the substrate (foam sheet) is preferably 3.000 mm or less, more preferably 2.000 mm or less and still more preferably 1.000 mm or less.

To improve stretchability and cushioning properties of the substrate, the density of the substrate is small. Therefore, the density of the substrate (foam sheet) is 0.4 g/cm$^3$ or less and preferably 0.35 g/cm$^3$ or less.

On the other hand, the density of the substrate is not too small from the standpoint of securing strength of the adhesive skin patch. Therefore, the density of the substrate (foam sheet) is 0.15 g/cm$^3$ or more, preferably 0.2 g/cm$^3$ or more and more preferably 0.25 g/cm$^3$ or more.

The stretchability of the substrate can be evaluated by various methods. For example, the stretchability can be evaluated using breaking strength and breaking elongation measured by the method described in the item of examples.

From the standpoint of the improvement of stretchability, the breaking strength of the substrate (foam sheet) is 20 N/20 mm or less, preferably 10 N/20 mm or less and more preferably 5 N/20 mm or less, and the breaking elongation thereof is 100% or more, preferably 200% or more and more preferably 250% or more.

To improve stretchability and cushioning properties of the substrate, a bubble fraction of the substrate is preferably high. Therefore, the bubble fraction of the substrate is preferably 50% or more, more preferably 60% or more and still more preferably 65% or more.

On the other hand, from the standpoint of securing the strength of the adhesive skin patch, the bubble fraction of the substrate (foam sheet) is preferably 85% or less, more preferably 80% or less and still more preferably 75% or less.

The material of the substrate (foam sheet) is not particularly limited, but is, for example, an amorphous crosslinking type. For example, the material includes polyurethane, polyolefin (such as polyethylene, polypropylene and their copolymers), silicone, acryl and rubber. Of those, rubber, polyethylene and polyurethane are preferred.

The production method of the substrate is not particularly limited, and for example, the substrate can be appropriately produced by the conventional methods.

Regarding the substrate, bubbles in the foam sheet may constitute an open cell structure or may constitute a closed cell structure. Among the bubbles in the foam sheet, bubbles that are connected with other bubbles are called open cell and bubbles that are not connected with other bubbles are called closed cell.

The adhesive layer (first adhesive layer) that is stuck to a skin contains the acrylic copolymer for the reasons that adhesive force to a skin is easy to be adjusted and skin irritation is less.

The acrylic copolymer is not particularly limited, but for example, an acrylic copolymer obtained from a monomer mixture containing a (meth)acrylic acid alkyl ester monomer and an alkoxy group-containing ethylenically unsaturated monomer is preferably used.

The (meth)acrylic acid alkyl ester monomer is a main component that imparts good skin stickability to the adhesive layer, and in particular, use of a long chain alkyl group having 6 or more carbon atoms, preferably 6 to 18 carbon atoms, in the alkyl moiety is effective. The (meth)acrylic acid alkyl ester monomer has the advantages that irritation to a skin is relatively small and the decrease of adhesive force is difficult to occur even by the use of a long period of time.

The (meth)acrylic acid alkyl ester monomer includes butyl ester, propyl ester, octyl ester, nonyl ester, decyl ester, dodecyl ester, lauryl ester and the like of acrylic acid or methacrylic acid, and those can be used in one kind or by combining two or more kinds. The alkyl ester chain thereof may be a straight chain and may be a branched chain.

The alkoxy group-containing ethylenically unsaturated monomer is a component that imparts water vapor permeability, so-called moisture permeability, to the acrylic copolymer. The alkoxy group-containing ethylenically unsaturated monomer is not particularly limited, but is preferably, for example, alkoxyalkyl acrylate having an alkoxy group with 1 to 4 carbon atoms, such as methoxypolyethylene glycol acrylate, ethoxydiethylene glycol acrylate, butoxydiethylene glycol acrylate, methoxyethyl acrylate, ethoxyethyl acrylate or butoxyethyl acrylate.

The acrylic copolymer is preferably an acrylic copolymer obtained from a monomer mixture containing a carboxyl group-containing ethylenically unsaturated monomer. The carboxyl group-containing ethylenically unsaturated monomer is a component that improves cohesive force of a copolymer obtained, and is useful in adjusting characteristics of the adhesive layer. Representative examples of the monomer include acrylic acid, itaconic acid, crotonic acid, fumaric acid and (anhydrous) maleic acid. Of those, acrylic acid is exemplified as the preferable monomer in the points of copolymerizability, handleability and the like.

The acrylic copolymer constituting the adhesive is preferably an acrylic copolymer obtained by copolymerization of the (meth)acrylic acid alkyl ester monomer and the alkoxy group-containing ethylenically unsaturated monomer and more preferably an acrylic copolymer obtained by copolymerization of the (meth)acrylic acid alkyl ester monomer, the alkoxy group-containing ethylenically unsaturated monomer and the carboxyl group-containing ethylenically unsaturated monomer.

In the acrylic copolymer, as necessary, in addition to the above-mentioned respective monomers, a monomer such as styrene, vinyl acetate or N-vinyl-2-pyrrolidone may be appropriately copolymerized as a modifier resin for performing various modifications such as incorporation of hydrophilicity.

The content of the (meth)acrylic acid alkyl ester monomer in the acrylic copolymer is preferably 40 to 80 mass % and more preferably 50 to 75 mass %. When the content of the (meth)acrylic acid alkyl ester monomer is in the above-mentioned range, the adhesive layer obtained shows good skin stickability, does not generate the phenomenon that an adhesive remains on the skin surface when peeling away from the skin, and has excellent peelability.

The content of the alkoxy group-containing ethylenically unsaturated monomer in the acrylic copolymer is preferably 10 to 60 mass %, more preferably 20 to 60 mass % and still more preferably 25 to 50 mass %. When the content of the alkoxy group-containing ethylenically unsaturated monomer is in the above-mentioned range, the resulting copolymer can impart excellent moisture permeability to the adhesive layer.

When the carboxyl group-containing ethylenically unsaturated monomer is contained, the content of the alkoxy group-containing ethylenically unsaturated monomer is preferably 10 to 50 mass % and more preferably 20 to 45 mass %.

The content of the carboxyl group-containing ethylenically unsaturated monomer is preferably 1 to 10 mass % and more preferably 3 to 8 mass %. When the content of the carboxyl group-containing ethylenically unsaturated monomer is in the above-mentioned range, the resulting copolymer can impart excellent cohesive force to the adhesive layer, can suppress the phenomenon of an adhesive residue and additionally can suppress skin irritation.

Weight average molecular weight of a soluble portion in a solvent for molecular weight measurement of the acrylic copolymer is preferably 500,000 to 2,500,000 and more preferably about 600,000 to 2,000,000.

The weight average molecular weight used herein is a value measured using gel permeation chromatography (GPC). A sample for measurement is dissolved in tetrahydrofuran, a soluble portion passing through a 0.45 $\mu$m$\phi$ membrane filter is subjected to GPC measurement and the value is calculated in terms of polystyrene.

The adhesive layer (first adhesive layer) that is stuck to a skin contains a liquid or pasty component at room temperature (25°C). The liquid or pasty component at room temperature is a component for imparting flexibility to the adhesive layer and reducing skin irritation when the adhesive layer has been brought into contact with the skin.

The liquid or pasty component at room temperature is not particularly limited so long as the component is compatible with the acrylic copolymer. Examples of the component that can be used include esters of phthalic acid, maleic acid, adipic acid, stearic acid or various fatty acids and alkyl alcohols, and esters of those acids and polyhydric alcohols such as ethylene glycol, glycerin or sorbitan. More specifically, examples of a monohydric alcohol ester include dibutyl phthalate, di-2-ethlhexyl phthalate, dibutyl adipate, di-2-ethylhexyl sebacate, dibutyl maleate, ethyl myristate, isopropyl myristate, isopropyl palmitate, butyl stearate, isopropyl isostearate, hexyl laurate, cetyl lactate, myristyl laurate, diethyl phthalate, octyldodecyl myristate, octyldodecyl oleate, hexyldecyl dimethyloctanoate, cetyl 2-ethylhexanoate, isocetyl 2-ethylhexanoate, stearyl 2-ethylhexanoate and dioctyl succinate. Examples of a dihydric or higher alcohol ester include propylene glycol dicaprylate, propylene glycol dicaprate, propylene glycol diisostearate, glyceryl monocaprylate, glyceryl tricaprylate, glyceryl tri-2-ethylhexanoate, glyceryl tricaprate, glyceryl trilaurate, glyceryl triisostearate, glyceryl trioleate, sorbitan trioeleate and trimethylolpropane tri-2-ethylheanoate. Those esters can be used in one kind or as mixtures of two or more kinds. From the standpoint of compatibility with the acrylic copolymer, the liquid or pasty component at room temperature is preferably carboxylic acid ester, more preferably glycerin fatty acid ester and still more preferably glycerin ester of saturated fatty acid.

Examples of the fatty acid include saturated fatty acids such as caprylic acid, capric acid and 2-ethylhexanoic acid. Those acids can be used alone or as combinations of two or more thereof. Those saturated fatty acids do not have an unsaturated double bond and therefore can prevent oxidative deterioration of the adhesive, that is, the adhesive layer.

When the above-described caprylic acid and the like are used as saturated fatty acids, glyceryl tricaplyrate, glyceryl tricaprate and glyceryl tri-2-ethylhexanoate are obtained as esters of various saturated fatty acids and alcohols. Of those carboxylic acid esters, glyceryl tricaprylate is particularly preferably used from the standpoint of compatibility with the acrylic copolymer, and the like.

The content of the liquid or pasty component at room temperature is, for example, preferably 20 to 100 parts by mass per 100 parts by mass of the acrylic copolymer. When the content of the component is in the above range, sufficient flexibility can be imparted to the adhesive layer. As a result, the adhesive layer has good stickability to a skin, suppresses physical skin irritation when peeling the patch from the skin and can achieve good peelability.

To improve adhesive force to a skin, the adhesive layer (first adhesive layer) that is stuck to the skin preferably further contains a tackifier. Rosin type tackifier, terpene type tackifier and the like can be used as the tackifier. Softening point of the tackifier is preferably 60°C or higher, more preferably 80°C or more and still more preferably 100°C or higher.

The content of the tackifier is, for example, preferably 5 to 50 parts by mass, more preferably 5 to 30 parts by mass and still more preferably 5 to 20 parts by mass, per 100 parts by mass of the acrylic copolymer.

As necessary, various additives such as the conventional plasticizer, softener, filler, stabilizer, antioxidant, antibacterial agent and fungicide may be appropriately added to the adhesive layer (first adhesive layer) that is stuck to a skin in a range that does not deviate the object of the present invention.

The adhesive layer (first adhesive layer) that is stuck to a skin is preferably subjected to a crosslinking treatment in order to impart appropriate cohesive force to the adhesive layer. The crosslinking treatment includes physical crosslinking by irradiation with ionizing radiation such as electron beams, $\gamma$ rays or X rays, and chemical crosslinking by a crosslinking agent. From the standpoints of handling properties and good reproducibility of crosslinking, the adhesive layer is preferably subjected to the chemical crosslinking treatment by a crosslinking agent. For example, a crosslinking agent is added to an adhesive composition having materials constituting an adhesive layer added thereto, and the resulting adhesive composition is heat-treated to perform a crosslinking treatment. Examples of such a crosslinking agent include crosslinking agents used in conducting a crosslinking treatment, such as an isocyanate crosslinking agent, a peroxide crosslinking agent or a metal chelate crosslinking agent.

The adhesive layer having been subjected to the crosslinking treatment as above holds a balance between appropriate skin stickability and cohesive force by the degree of crosslinking, but a molecular weight and molecular weight distribution of the acrylic copolymer also affect the adjustment of those.

The adhesive layer (first adhesive layer) that is stuck to a skin is preferably thick from the standpoint of improvement of adhesive force. However, when too thick, the edge of the adhesive layer is caught and is easy to be peeled or the adhesive layer is easy to protrude from the edge and the edge is easy to be contaminated, during the adhesive layer being stuck to a skin.

For the above reasons, the thickness of the adhesive layer (first adhesive layer) is preferably 50 $\mu$m or more and more preferably 60 $\mu$m or more. On the other hand, the thickness is preferably 120 $\mu$m or less and more preferably 100 $\mu$m or less.

The adhesive layer (first adhesive layer) may be formed on the entire surface of the substrate or may be partially formed on the surface thereof. From the standpoint of air permeability, the adhesive layer is preferably partially formed. The shape when partially forming the adhesive layer is not particularly limited. For example, the adhesive layer may have a given pattern shape such as a lattice shape, a line shape (such as linear shape or wavy line shape), or a dot shape (point shape or island shape).

In case where the adhesive skin patch of the present embodiment has the adhesive layer (first adhesive layer) partially formed on one side (first surface) of the substrate and another adhesive layer (second adhesive layer) formed on the other side (second surface) of the substrate, the thickness of the second adhesive layer is not particularly limited, but is, for example, 30 to 80 $\mu$m and preferably 40 to 70 $\mu$m.

In this case, the composition of the second adhesive layer may be the same as or different from the composition of the first adhesive layer described above. For example, the second adhesive layer may contain or may not contain the acrylic copolymer and the liquid or pasty component at room temperature. Furthermore, other than the acrylic adhesive, a rubber adhesive, a urethane adhesive, a silicone adhesive or the like can be used as the adhesive forming the second adhesive layer.

The second adhesive layer may be formed on the whole of the other side (second surface) of the substrate or may be partially formed on the second surface.

The adhesive skin patch of the present embodiment may be provided with the substrate described above and a layer other than the adhesive layer so long as the effect of the present invention is exerted.

In the adhesive skin patch of the present embodiment, the surface of the adhesive layer is preferably protected until using the adhesive skin patch for the purpose of preventing the surface of the adhesive layer from being contaminated. The protection method of the adhesive includes a method of protecting the adhesive by laminating a release liner on the adhesive layer. In case where only one side of the adhesive skin patch is an adhesive surface, the adhesive layer is protected by that the surface of the substrate on which the adhesive layer is not formed is subjected to a release treatment and the adhesive skin patch is wound such that the adhesive layer is laminated on the release-treated surface. In addition, in case where both sides of the adhesive skin patch are an adhesive surface, both adhesive layers may be protected by that one release-treated surface of a release liner, both sides of which having a release-treated surface, is laminated on one adhesive surface and the adhesive skin patch is wound such that the other release-treated surface of the release liner is brought into contact with the other adhesive surface.

<Production method>

Production method of the adhesive skin patch of the present embodiment is not particularly limited. For example, the adhesive layer and the substrate are separately formed, and then those are laminated to each other. Alternatively, a solution of the adhesive is applied to the substrate and dried, thereby forming the adhesive layer.

Examples

The effects of the present invention are described in detail below by reference to examples and comparative examples, but the present invention is not construed as being limited to those. All "parts" and "%" in the following examples and comparative examples mean "parts by mass" and "mass %", respectively.

[Production of substrate]

(Substrate 1)

2.88 g of a vulcanization accelerator (trade name: CLFB-2, manufactured by E-TEC) was added to 75g of a styrene-butadiene copolymer mixture (trade name: AH748-M4, manufactured by E-TEC), the resulting mixture was stirred in a rotational speed of 1800 rpm for 5 minutes using a stirrer. The solution thus obtained was applied to the release-treated surface of a release-treated polyethylene terephthalate film (trade name: DIAFOIL MRF38, manufactured by Mitsubishi Chemical Corporation) such that the thickness after drying became 0.325 mm, and then dried at 130°C for 10 minutes. The coated surface was subjected to a plasma treatment to obtain a substrate 2. The substrate 2 was a porous sheet having open cells.

(Substrate 2)

A polypropylene foam sheet (trade name: FOLEC OPN 1.0t, manufactured by INOAC Corporation) was prepared as a substrate 2. The substrate 2 was a porous sheet having open cells.

(Substrate 3)

A polyethylene terephthalate nonwoven fabric sheet (trade name: SONTARA #8010, manufactured by Du Pont) was prepared as a substrate 3.

(Substrate 4)

A polyurethane film (trade name: ESMER URS-210, manufactured by Nihon Matai Co., Ltd.) was subjected to a corona treatment to obtain a substrate 4.

(Substrate 5)

A polyethylene terephthalate film (trade name: DIAFOIL T100-38, manufactured by Mitsubishi Chemical Corporation) was prepared as a substrate 5.

(Substrate 6)

A polyethylene foam sheet (trade name: VOLARA XL-H #03001 (80), manufactured by Sekisui Chemical Co., Ltd.) was subjected to a corona treatment to obtain a substrate 6. The substrate 6 was a porous sheet that does not have open cells and has only closed cells.

[Evaluation of substrate]

Thickness, density, bubble fraction, breaking strength and breaking elongation of the substrates 1 to 6 obtained above were measured as follows.

(Thickness)

Thicknesses at 5 points of each substrate were measured using a dial gauge, and an average value of the measurement results was used as a thickness of each substrate.

(Density)

Each substrate was cut into a size of 5 cm $\times$ 5 cm, and its weight was measured. Density was calculated by the following formula using the weight measured and the above thickness.

$$\text{Density (g/cm}^3) = \text{Weight (g)}/(5 \times 5 \times \text{thickness (cm}^3))$$

(Bubble fraction)

Bubble fraction of each substrate was calculated by the following formula.

$$\text{Bubble fraction (\%)} = (1 \times \text{specific gravity of substrate material} - \text{density}) \times 100$$

(Breaking strength and breaking elongation)

Each substrate was cut into a size of 20 mm width and 100 mm length, set up on a tensile tester (trade name: AUTOGRAPH AG-IS, manufactured by Shimadzu Corporation) in a chuck distance of 40 mm and pulled in a tensile speed of 300 mm/min. Load and elongation when the substrate broke were measured. The same measurement was conducted three times, and its average value was used as breaking strength and breaking elongation of each substrate.

[Production of adhesive skin patch]

(Example 1)

30 parts of glyceryl tricaprylate, 10 parts of a rosin ester (HARITACK PCJ, manufactured by Harima Chemicals Group, Inc.) as a tackifier and 0.05 parts of a trifunctional isocyanate compound (trade name: CORONATE HL, manufactured by Tosoh Corporation) as a crosslinking agent were blended with 100 parts of an acrylic copolymer (copolymer comprising isononyl acrylate : 2-methoxyethyl acrylate : acrylic acid = 65 parts : 30 parts : 5 parts) in toluene, and a uniform adhesive solution (adhesive A) was prepared.
The adhesive solution was applied to a release-treated surface of a release paper (trade name: KP-64 SHIROMARU D MARUMIZU, manufactured by LINTEC Corporation) such that a thickness after drying became 70 μm, and a wavy groove of width: 0.5 mm, amplitude: 50 mm, period: 140 mm and pitch: 5 mm was provided, followed by drying at 130°C for 3 minutes. Thus, an adhesive layer was formed. The adhesive layer formed was transferred to and laminated on a plasma-treated surface of the substrate 1, followed by allowing to stand at 60°C for 72 hours. Thus, an adhesive skin patch of Example 1 was obtained.

(Example 2 and Comparative Examples 1 to 5)

Adhesive skin patch of Example 2 was obtained in the same manner as in Example 1, except for using an adhesive solution (adhesive B) obtained in the same manner as in the adhesive A except that the amount of glyceryl tricaprylate added was changed to 20 parts by mass and the amount of the trifunctional isocyanate compound added was changed to 0.07 parts by mass.

Adhesive skin patch of Comparative Example 1 was obtained in the same manner as in Example 1, except that the substrate 2 was used in place of the substrate 1.

Adhesive skin patch of Comparative Example 2 was obtained in the same manner as in Example 1, except that the substrate 3 was used in place of the substrate 1.

Adhesive skin patch of Comparative Example 3 was obtained in the same manner as in Example 1, except that the substrate 4 was used in place of the substrate 1 and the adhesive layer was formed on the corona-treated surface of the substrate 4.

Adhesive skin patch of Comparative Example 4 was obtained in the same manner as in Example 1, except that the substrate 5 was used in place of the substrate 1.

Adhesive skin patch of Comparative Example 5 was obtained in the same manner as in Example 1, except that the substrate 6 was used in place of the substrate 1 and the adhesive layer was formed on the corona-treated surface of the substrate 6.

[Evaluation of adhesive skin patch]

Stress relaxation percentage of the adhesive skin patch of each example and comparative example was measured as follows. Additionally, feeling of sticking and long-term stickability were measured. The results are shown in Table 1.

(Stress relaxation percentage)

The adhesive skin patch of each example and comparative example was stuck to a measurement stage, and energy E1 that the stage receives when a ball of 8.9 g was dropped on the stage from a height of 25 cm was measured. Energy E0 was measured in the same manner as above, except that the adhesive skin patch was not stuck to the measurement stage. Stress relaxation percentage was calculated from those values by the following formula (1).

$$\text{Stress relaxation percentage (\%)} = [(E0 - E1)/E1] \times 100 \qquad (1)$$

(Feeling of sticking)

Adhesive skin patch of each example and comparative example was cut into a size of 10 mm $\times$ 50 mm, and a test sample was produced.

The adhesive layer of the test sample from which a release paper had been peeled was stuck to a joint part of an index finger of a participant. The participant bent his index finger and the feeling of sticking of the patch was evaluated by the following criteria.

5: No feeling of tightness
4: Feeling of tightness in a degree that patch is not annoying
3: Feeling of tightness in a degree that patch is slightly annoying, but participant can live life
2: Feeling of tightness in a degree that patch is annoying, but participant can endure and live life without peeling patch
1: Feeling of tightness in a degree that participant cannot endure without peeling patch

(Long-term stickability)

Each adhesive skin patch of example and comparative example was cut into a circular shape having a diameter of 35 mm, and a weight having a diameter of 30 mm and a thickness of 5 mm was bonded to the surface of the substrate side of the patch using a double-sided tape (trade name: No. 5000NS, manufactured by Nitto Denko Corporation). Thus, a test sample was obtained. The adhesive layer of the test sample was stuck to an upper arm of a participant, and the period until 80% or more of the portion protruding from the weight of the adhesive skin patch is peeled from the time the adhesive layer was stuck was measured. The measurement period is up to 7 days, and the test sample that 80% or more of the portion protruding from the weight of the adhesive skin patch is not peeled at the time that 7 days are expired is shown as "7 days <" in Table 1.

[Table 1]

| | | Example 1 | Example 2 | Comp. Example 1 | Comp. Example 2 | Comp. Example 3 | Comp. Example 4 | Comp. Example 5 |
|---|---|---|---|---|---|---|---|---|
| Substrate | Material | SBR | SBR | PP | PET | PU | PET | PE |
| | Form | Foam | Foam | Foam | Non-woven fabric | Film | Film | Foam |
| | Open cell | Presence | Presence | Presence | - | - | - | None |
| | Thickness (mm) | 0.325 | 0.325 | 0.920 | 0.280 | 0.030 | 0.038 | 0.110 |
| | Density (g/cm$^3$) | 0.257 | 0.257 | 0.035 | 0.156 | 1.220 | 1.350 | 0.271 |
| | Bubble Fraction | 74% | 74% | 97% | 84% | 0% | 0% | 73% |
| | Breaking strength (N/20 mm) | 1.34 | 1.34 | 4.8 | 40.7 | 18.7 | 145.0 | 9.5 |
| | Breaking elongation (%) | 374 | 374 | 110 | 57 | 1019 | 133 | 480 |
| Adhesive layer | Kind of Adhesive | A | B | A | A | A | A | A |
| Evaluation of patch | Stress relaxation percentage (%) | 41.8 | 41.8 | 52.0 | 11.4 | 13.3 | 10.0 | 26.5 |
| | Feeling of Sticking | 5 | 5 | 4 | 2 | 5 | 1 | 4 |
| | Long-term stickability | 7 days < | 7 days < | 7 days < | 4 days | 7 days < | 3 days | 7 days < |

SBR: Styrene-butadiene rubber
PP: Polypropylene
PET: Polyethylene terephthalate
PU: Polyurethane
PE: Polyethylene

In Comparative Example 2, non-woven fabric having small thickness, large breaking strength and small breaking elongation was used as the substrate. The adhesive skin patch of Comparative Example 2 had low stress relaxation percentage (cushioning properties). Therefore, when a wearable device has been mounted on a skin using this patch, skin irritation is large. Additionally, the adhesive skin patch of Comparative Example 2 was that evaluation results of feeling of sticking and long-term stickability were poor.

In Comparative Example 3, urethane film having small thickness and large density was used as the substrate. The adhesive skin patch of Comparative Example 3 showed low relaxation percentage (cushioning properties). Therefore, when a wearable device has been mounted on a skin using this patch, skin irritation is large.

In Comparative Example 4, polyethylene terephthalate film having small thickness, large breaking strength and large density was used as the substrate. The adhesive skin patch of Comparative Example 4 showed low stress relaxation percentage (cushioning properties). Therefore, when a wearable device has been mounted on a skin using this patch, skin irritation is large. Additionally, the adhesive skin patch of Comparative Example 3 was that evaluation results of feeling of sticking and long-term stickability were poor.

In Comparative Example 5, the thickness of the substrate is not sufficient. Therefore, stress relaxation percentage (cushioning properties) is insufficient, and when a wearable device has been mounted on a skin using this patch, there is a possibility that impression or the like occurs on a skin surface by the wearable device.

On the other hand, the adhesive skin patches of Examples 1 and 2 showed high stress relaxation percentage (cushioning properties). Therefore, when a wearable device has mounted on a skin using those patches, skin irritation is small. Additionally, regarding the adhesive skin patches of Examples 1 and 2, evaluation results of feeling of sticking and long-term stickability were good, and inflammation did not occur on a skin surface.

Although preferred embodiments of the present invention have been described, the present invention is not limited to the embodiments described above, and various modifications or substitutions can be added to the embodiments described above in the scope that the scope of the present invention does not deviate.

Reference Signs List

1, 11, 21: Adhesive skin patch
2, 12, 22: Substrate
3, 13, 23: Adhesive layer (first adhesive layer)
14: Other adhesive layer (second adhesive layer)
15, 25: Skin
16,26: Wearable device

**Claims**

1. An adhesive skin patch comprising a substrate and an adhesive layer,

    wherein the substrate is formed of a foam sheet having breaking elongation of 100% or more as measured according to the method of the description, breaking strength of 10 N/20 mm or less as measured according to the method of the description, a density in the range of from 0.15 to 0.4 g/cm$^3$ and a thickness of 0.300 mm or more, the adhesive layer is formed on one side of the substrate, and the adhesive layer contains an acrylic copolymer and a liquid or pasty component at room temperature.

2. The adhesive skin patch according to claim 1, wherein another adhesive layer is provided on a surface on a side opposite the surface of the substrate having the adhesive layer.

3. The adhesive skin patch according to claim 1 or 2, wherein the liquid or pasty component at room temperature contains a carboxylic acid ester.

4. The adhesive skin patch according to claim 3, wherein the carboxylic acid ester contains a glycerin ester of a saturated fatty acid.

**Patentansprüche**

1. Hautpflaster, bestehend aus einem Substrat und einer Klebeschicht,

    wobei das Substrat aus einer Schaumstoffplatte mit einer Bruchdehnung von mindestens 100 %, einer gemäß dem Verfahren der Beschreibung gemessenen Bruchfestigkeit von maximal 10 N/20 mm, einer Dichte im Bereich von 0,15 bis 0,4 g/cm$^3$ und einer Dicke von mindestens 0,300 mm besteht, die Klebeschicht auf einer Seite des Substrats aufgebracht ist und die Klebeschicht ein Acrylcopolymer und eine bei Raumtemperatur flüssige oder pastöse Komponente enthält.

2. Hautpflaster nach Anspruch 1, wobei eine weitere Klebeschicht auf der der Oberfläche des Substrats mit der Klebeschicht gegenüberliegenden Seite aufgebracht ist.

3. Hautpflaster nach Anspruch 1 oder 2, wobei die bei Raumtemperatur flüssige oder pastöse Komponente einen Carbonsäureester enthält.

4. Hautpflaster nach Anspruch 3, wobei der Carbonsäureester einen Glycerinester einer gesättigten Fettsäure enthält.

**Revendications**

1. Timbre cutané adhésif comprenant un substrat et une couche adhésive,

    dans lequel le substrat est formé sur une feuille de mousse ayant un allongement à la rupture de 100 % ou plus tel que mesuré selon le procédé de la description, une résistance à la rupture de 10 N/20 mm ou inférieure telle que mesurée selon le procédé de la description, une densité dans la plage de 0,15 à 0,4 g/cm$^3$ et une épaisseur de 0,300 mm ou plus,
    la couche adhésive est formée sur un côté du substrat, et
    la couche adhésive contient un copolymère acrylique et un composant liquide ou pâteux à la température ambiante.

2. Timbre cutané adhésif selon la revendication 1, dans lequel une autre couche adhésive est disposée sur une surface d'un côté opposé à la surface du substrat ayant la couche adhésive.

3. Timbre cutané adhésif selon la revendication 1 ou 2, dans lequel le composant liquide ou pâteux à la température ambiante contient un ester d'acide carboxylique.

4. Timbre cutané adhésif selon la revendication 3, dans lequel l'ester d'acide carboxylique contient un ester de glycérine d'un acide gras saturé.

*FIG. 1*

*FIG. 2*

*FIG. 3*

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2015530225 W
- EP 0676183 A1
- EP 3498245 A1
- EP 2799472 A1
- JP H045807 U